(19) **Europäisches Patentamt / European Patent Office / Office européen des brevets**

(11) **EP 1 818 047 A2**

(12) # EUROPÄISCHE PATENTANMELDUNG

(43) Veröffentlichungstag:
**15.08.2007 Patentblatt 2007/33**

(51) Int Cl.:
*A61K 9/16* (2006.01)     *A61K 9/50* (2006.01)

(21) Anmeldenummer: **07108392.7**

(22) Anmeldetag: **04.03.2003**

(84) Benannte Vertragsstaaten:
**AT BE BG CH CY CZ DE DK EE ES FI FR GB GR HU IE IT LI LU MC NL PT RO SE SI SK TR**

(30) Priorität: **07.03.2002 DE 10209982**

(62) Dokumentnummer(n) der früheren Anmeldung(en) nach Art. 76 EPÜ:
**03722336.9 / 1 499 298**

(71) Anmelder: **Boehringer Ingelheim Pharma GmbH & Co. KG**
**55216 Ingelheim am Rhein (DE)**

(72) Erfinder: **Die Erfindernennung liegt noch nicht vor**

Bemerkungen:
Diese Anmeldung ist am 16 - 05 - 2007 als Teilanmeldung zu der unter INID-Kode 62 erwähnten Anmeldung eingereicht worden.

(54) **Oral zu applizierende Darreichungsform für schwerlösliche basische Wirkstoffe**

(57)     Die Erfindung betrifft eine neue, oral zu applizierende Darreichungsform für Wirkstoffe mit pH-abhängigen Löslichkeitsverhalten und deren pharmakologisch verträglichen Salze, welche die Bioverfügbarkeit des Wirkstoffs verbessert.

EP 1 818 047 A2

## Beschreibung

Die Erfindung betrifft eine oral zu applizierende Darreichungsform für basische Wirkstoffe mit einem pH-abhängigen Löslichkeitsverhalten und deren Salze.

Als "Wirkstoff" im Sinne dieser Erfindung wird jede pharmakologisch wirksame Verbindung angesehen, die (als solche oder als eines ihrer pharmazeutisch annehmbaren Salze) eine schwache Base ist und im Bereich von pH 1 bis pH 7,5 ein pHabhängiges Löslichkeitsverhalten (mit besserer Löslichkeit unter sauren Bedingungen und schlechterer Löslichkeit unter basischen Bedingungen) zeigt. Bei solchen Wirkstoffen kann nämlich die Bioverfügbarkeit bei oraler Gabe von dem pH-Wert im Magen-Darm-Trakt abhängig sein. Vorzugsweise weisen Wirkstoffe im Sinne dieser Erfindung in wässrigen Lösungen bei niedrigen pH-Werten durch ein- oder mehrfache Protonierung verhältnismäßig hohe Sättigungslöslichkeiten auf, während die neutralen Verbindungen bei pH-Werten über 5 gemäß der Definition des europäischen Arzneibuches praktisch unlöslich sind (Sättigungslöslichkeit < 100 µg / ml).

Die erfindungsgemäße orale Darreichungsform kann als Wirkstoff beispielsweise 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester (WO98/37075), BIBU 104 (Lefradafiban; (3*S*,5*S*)-5-[[4'-(*N*-Methoxycarbonylamidino)-4-biphenylyl]-oxymethyl]-3-[(methoxycarbonyl)methyl]-2-pyrrolidinon; EP 0 483 667), (*R*)-2-[4-(*N*-Phenylcarbonylamidino)-phenylaminomethyl]-1-methyl-5-[1-(*n*-propyloxycarbonylmethylamino)-1-(pyrrolidinocarbonyl)-ethyl]-benzimidazol (WO 01/47896), Telmisartan, DTTX 30 SE, BIBV 308 SE (Terbogrel), Bromhexin, BIIL 284 (Amelubant; 4-((3-((4-(1-(4-Hydroxyphenyl)-1-methylethyl)phenoxy)methyl)benzyl)-oxy)benzenecarboximidamid-N-Ethylcarboxylat; WO 96/02497), Flibanserin (1-[2-(4-(3-Trifluoromethyl-phenyl)piperazin-1-yl)ethyl]-2,3-dihydro-1H-benzimidazol-2-on; EP-A-526434;), 4-(4-(2-Pyrrolylcarbonyl)-1-piperazinyl)-3-trifluormethyl-benzoyl-guanidin (WO 00/17176), Pimobendan oder eines der pharmakologisch verträglichen Salze dieser Verbindungen (wie etwa Hydrochloride, Hydrobromide, Mesylate, Sulfate und Phosphate) enthalten.

Die Löslichkeit einer Verbindung kann bestimmt werden, indem man einen Überschuss der Verbindung bei Raumtemperatur in dem jeweiligen Medium dispergiert und für einen definierten Zeitraum (ca. 1 bis 24 h) bis zur Einstellung des Gleichgewichts kräftig schüttelt. Nach Filtration wird in dem klaren Filtrat der pH-Wert und mittels Spektralphotometrie oder einem anderen geeigneten analytischen Verfahren die Konzentration der gelösten Substanz bestimmt.

Das pH-abhängige Löslichkeitsverhalten des Wirkstoffes kann je nach Dosis dazu führen, dass dieser bei oraler Verabreichung konventionell zusammengesetzter fester Arzneiformen nur dann im Magen des Patienten vollständig in Lösung gehen kann, wenn die im Magen vorhandene Flüssigkeit einen hinreichend niedrigen pH-Wert aufweist. Ist der pH-Wert im Magen erhöht (dies kann durch normale physiologische Variabilität, durch eine Krankheit oder durch eine Komedikation mit solchen Arzneimittel bedingt sein, die den Magen-pH erhöhen), kann der Wirkstoff nicht mehr vollständig in Lösung gehen.

Der Einfluß der Dosis des jeweiligen Wirkstoffs auf seine Bioverfügbarkeit läßt sich quantitativ mittels des Konzepts der (dimensionslosen) Dosenumber (Do) beschreiben. Die Dosenumber wird definiert als:

$$Do = (Mo / Vo) / Cs \, ,$$

wobei

Mo = Dosis (mg),
Vo = vorhandenes Flüssigkeitsvolumen (ml) und
Cs = Sättigungslöslichkeit (mg / ml).

Gemäß einer heute üblichen Annahme beträgt das Flüssigkeitsvolumen im Magen nach Einnahme einer Darreichungsform ca. 250 ml. (Löbenberg, R., Amidon, G.L.: Modern bioavailability, bioequivalence and biopharmaceutics classification system. New scientific approaches to international regulatory standards (Eur. J. Pharm. Biopharm. 50 (2000) 3-12).

Bei Dosierungen, die eine Dosenumber von kleiner 1 ergeben, treten keine Löslichkeitsprobleme auf. Erst wenn die kritische Dosenumber von 1 überschritten wird, kann es zu relevanten Verschlechterungen des Löslichkeit und damit zur Abnahme der Bioverfügbarkeit kommen. In der Regel beginnt die eigentliche Problemzone jedoch erst bei Dosierungen, die eine Dosenumber deutlich höher als 1 ergeben, da zumindest Teile der gelösten Substanz durch den Resorptionsvorgang ständig aus dem Gleichgewicht entfernt werden.

Die in der erfindungsgemäßen oralen Darreichungsform enthaltenen Wirkstoffe weisen für die Dosenumber bezogen auf die Löslichkeit bei pH < 2 (d.h. genügend saurer Magen) einen Wert von unter 1 und für die Dosenumber bezogen auf die Löslichkeit bei pH > 5 (d.h. keine oder nur verschwindend wenig Magensäure) einen Wert von deutlich über 1 auf, d.h. für die erfindungsgemäße orale Darreichungsform ist sowohl das Ausmaß der pH-Abhängigkeit der Löslichkeit des Wirkstoffs als auch die Höhe der Wirkstoffdosis von Interesse.

Eine Anhebung des Magen-pH-Wertes kann bei Wirkstoffen mit einer derartigen Löslichkeitscharakteristik dann in einer erheblich verminderten Bioverfügbarkeit des Wirkstoffes (bis hin zu einer vollständigen Malabsorption) führen, die unter Umständen in einem Therapieversagen resultiert. Eine generelle Dosiserhöung zur Kompensation der verminderten Bioverfügbarkeit in Patienten mit erhöhtem Magen-pH ist wegen der Wirkstoffverschwendung sowie wegen der erhöhten Belastung des Patienten und des damit verbundenen Risikos z.B. von Nebenwirkungen häufig unerwünscht bzw. aufgrund der Arzneimittelsicherheit überhaupt nicht möglich.

Aufgabe der Erfindung ist es, eine oral zu applizierende pharmazeutische Zusammensetzung für Wirkstoffe mit pH-abhängigen Löslichkeitsverhalten bereitzustellen, die die weitgehend pH-unabhängige Bioverfügbarkeit des Wirkstoffs gewährleistet.

Überraschenderweise wurde nun gefunden, dass der Einsatz pharmazeutisch akzeptabler organischer Säuren mit einer Wasserlöslichkeit von > 1 g / 250 ml bei 20° C, vorzugsweis von > 1 g /160 ml bei 25 °C, in festen oralen Darreichungsformen eine hinreichende Bioverfügbarkeit von Wirkstoffen mit pH-abhängigen Löslichkeitsverhalten auch an Patienten mit erhöhtem Magen-pH gewährleisten kann.

Pharmazeutisch geeignete Säuren im Sinne dieser Erfindung sind beispielsweise Weinsäure, Fumarsäure, Bernsteinsäure, Zitronensäure, Apfelsäure, Glutaminsäure und Asparaginsäure einschließlich deren Hydraten und sauren Salzen. Besonders geeignet im Sinne dieser Erfindung sind Weinsäure, Fumarsäure, Bernsteinsäure und Zitronensäure, insbesondere Weinsäure, Fumarsäure und Bernsteinsäure.

Zahlreiche Wirkstoffe zeigen in Gegenwart von Säuren und Spuren von Wasser eine mehr oder weniger stark ausgeprägte Neigung zu hydrolytischer Zersetzung. In Einzelfällen kann es auch zu einer direkten chemischen Umsetzung zwischen dem Wirkstoff und organischen Säuren, z.B. zu einer Esterbildung, kommen. Zur Entwicklung eines lagerstabilen Produktes ist es daher vorteilhaft, in der Darreichungsform die organische Säure räumlich vom Wirkstoff zu trennen. Erst nach Applikation der Darreichungsform geht dann die organische Säure in Lösung und erzeugt ein saures Mikroklima, in dem sich der Wirkstoff auflösen kann.

Eine weitere Aufgabe der Erfindung besteht daher darin, die unerwünschten Wechselwirkungen zwischen Säure und Wirkstoff trotz Verwendung einer organischen Säure zur Löslichkeitsverbesserung zu vermeiden.

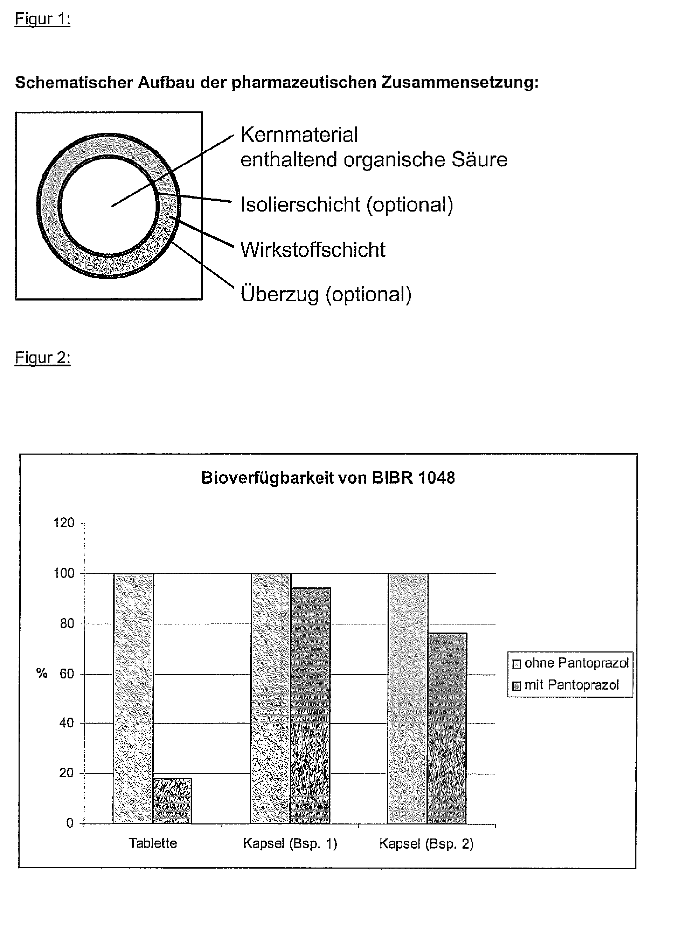

Für die bevorzugte räumliche Trennung von Wirkstoff und organischer Säure sind multipartikuläre Darreichungsformen, in denen die einzelnen Partikel wie in Figur 1 aufgebaut sind, besonders geeignet.

Figur 1 stellt den schematischen Aufbau der pharmazeutischen Zusammensetzung anhand eines Schnitts durch ein für die Herstellung der erfindungsgemäßen pharmazeutischen Zusammensetzung geeignetes Pellet dar. Der annähend kugelförmige/sphärische Kernbereich dieses Pellets enthält/besteht aus der pharmazeutisch akzeptablen organischen Säure. Dann folgt gegebenenfalls eine Schicht, die den Säure-Kern von der wirkstoffhaltigen Schicht trennt, die sog. Isolierschicht. Die Isolierschicht wiederum, bzw. das Kernmaterial in Abwesenheit einer Isolierschicht, ist von der ebenfalls kugelschalenförmigen Wirkstoffschicht umgeben, die ihrerseits von einem Überzug umschlossen sein kann, der die Abriebfestigkeit und die Lagerstabilität der Pellets erhöht.

Als Kernmaterial wird eine pharmazeutisch akzeptable organische Säuren mit einer Wasserlöslichkeit von > 1 g / 250 ml bei 20° C, wie z.B. Weinsäure, Fumarsäure, Bernsteinsäure, Zitronensäure, Apfelsäure, Glutaminsäure und Asparaginsäure einschließlich deren Hydraten und sauren Salzen, verwendet, der gegebenenfalls ein geringer Anteil von 1 bis 10 Gew.-%, bevorzugt 3 bis 6 Gew.-% eines geeignete Bindemittels zugesetzt wird. Die Verwendung eines Bindemittels ist z.B. dann erforderlich, wenn das Kernmaterial in einem Kessel-aufbau-verfahren hergestellt wird. Bei Verwendung eines Extrusions- oder Sphäronisierungs-Verfahrens benötigt man an Stelle von Bindemitteln andere technologische Hilfsstoffe, beispielsweise mikrokristalline Cellulose. Es ist auch denkbar, reine (100 % ige) Säure als Startmaterial zu verwenden, wenn man diese in hinreichend enger Korngrößenverteilung beschaffen kann. Als pharmazeutisch akzeptable organische Säure werden bevorzugt Weinsäure, Fumarsäure, Bernsteinsäure oder Zitronensäure eingesetzt; besonders bevorzugt ist Weinsäure. Als Bindemittel können Gummi arabicum oder ein partial- oder vollsynthetischem Polymer aus der Gruppe der Hydroxypropylcellulosen, der Hydroxypropylmethylcellulosen, der Methylcellulosen, der Hydroxyethylcellulosen, der Carboxymethylcellulosen, der Polyvinylpyrrolidone, der Copolymerisate aus *N*-Vinylpyrrolidon und Vinylacetat, oder Kombinationen dieser Polymere verwendet werden; bevorzugt ist Gummi arabicum. Das sphärische Kernmaterial hat vorzugsweise einen mittleren Durchmesser von 0,4 - 1,5 mm. Der Gehalt der pharmazeutisch akzeptablen organischen Säure liegt üblicherweise zwischen 30 und 100 % im Kernmaterial.

Zur Erhöhung der Lagerstabilität des Fertigproduktes ist es vorteilhaft, das Kernmaterial vor dem Auftrag des Wirkstoffes mit einer Isolierschicht basierend auf einem wasserlöslichen, pharmazeutisch akzeptablen Polymer zu beschichten. Als solches wasserlösliches Polymer kommen beispielsweise Gummi arabicum oder ein partial- oder vollsynthetischem Polymer aus der Gruppe der Hydroxypropylcellulosen, der Hydroxypropylmethylcellulosen, der Methylcellulosen, der Hydroxyethylcellulosen, der Carboxymethylcellulosen, der Polyvinylpyrrolidone, der Copolymerisate aus N-Vinylpyrrolidon und Vinylacetat, oder Kombinationen dieser Polymere in Frage. Bevorzugt wird Gummi arabicum oder eine Hydroxypropylmethylcellulose verwendet. Gegebenenfalls kann die Beschichtung mit dem wasserlöslichen, pharmazeutisch akzeptablen Polymer unter Zusatz geeigneter Weichmacher, Trennmittel und Pigmente erfolgen, wie beispielsweise Triethylcitrat, Tributylcitrat, Triacetin, Polyethylenglykole (Weichmacher), Talkum, Kieselsäure (Trennmittel), Titandioxid oder Eisenoxidpigmente (Pigmente).

Die Wirkstoffschicht enthält den Wirkstoff sowie Binde- und gegebenenfalls Trennmittel. Als Bindemittel können beispielsweise Hydroxypropylcellulose, Hydroxypropylmethylcellulose, Methylcellulose, Hydroxyethylcellulose, Carboxymethylcellulose, Polyvinylpyrrolidon, Copolymerisate aus *N*-Vinylpyrrolidon und Vinylacetat oder Kombinationen dieser Polymere verwendet werden. Vorzugsweise wird Hydroxypropylcellulose oder Copolymerisate aus *N*-Vinylpyrrolidon und Vinylacetat eingesetzt. Der Zusatz von Trennmitteln wie z.B. Talkum, Magnesiumstearat oder Kieselsäure dient dazu, ein Zusammenwachsen der Partikel während des Prozesses zu verhindern. Der bevorzugte Wirkstoffgehalt beträgt maximal 60 %, vorzugsweise maximal 50 % der pharmazeutischen Zusammensetzung.

Die optionale äußerste Schicht, die zur Verminderung eines erhöhten Abriebs bei der Abfüllung in Kapseln und/oder zur Erhöhung der Lagerstabilität dient, besteht aus pharmazeutisch üblichen Filmbildnern, Weichmachern und gegebenenfalls Pigmenten. Als Filmbildner können beispielsweise Hydroxypropylcellulose, Hydroxypropylmethylcellulose, Methylcellulose, Polymerisate und Copolymerisate der Acryl- und Methacrylsäure und deren Estern, oder Kombinationen dieser Polymere verwendet werden. Als Weichmacher kommen unter anderem Triethylcitrat, Tributylcitrat, Triacetin oder Polyethylenglykole in Frage. Als Pigmente können z.B. Titandioxid oder Eisenoxidpigmente eingesetzt werden. Bevorzugt besteht der äußere Überzug aus Hydroxypropylmethylcellulose und / oder Methylcellulose, gegebenenfalls unter Zusatz von Polyethylenglykolen als Weichmacher.

Die Pellets können nach dem im folgenden beschriebenen Verfahren hergestellt werden:

Das säurehaltige Kernmaterial besteht entweder aus Kristallen der jeweils zur Verwendung kommenden organischen Säure oder vorteilhafter aus annähernd sphärischen Partikeln in der gewünschten Größe mit einem hohen Gehalt an organischer Säure, die mittels Verfahren hergestellt werden können, die in der pharmazeutischen Technologie bekannt und etabliert sind. In Frage kommen insbesondere der Aufbau des Kernmaterials in Kesselverfahren, auf Pelletiertellern, oder mittels Extrusion /Sphäronisierung. Anschließend kann das so erhaltenen Kernmaterial durch Sieben in Fraktionen mit dem gewünschten Durchmesser geteilt werden. Geeignetes Kernmaterial hat einen mittleren Durchmesser von 0,4 bis 1,5 mm, bevorzugt von 0,6 bis 0,8 mm.

Auf dieses säurehaltige Kernmaterial wird zunächst die Isolierschicht aufgetragen. Dies kann durch übliche Verfahren, z.B. durch Auftragen einer wäßrigen Dispersion des wasserlöslichen, pharmazeutisch akzeptablen Polymers gegebenenfalls unter Zusatz von Weichmachern, Trennmitteln und / oder Pigmenten in der Wirbelschicht, in Dragierkesseln oder in üblichen Filmcoatinganlagen, geschehen. Falls erforderlich, kann anschließend erneut gesiebt werden.

Daran anschließend wird der Wirkstoff aus einer bindemittel- und ggf. trennmittelhaltigen Dispersion aufgetragen. Das flüchtige Dispersionsmittel wird während des Prozesses und / oder daran anschließend durch Trocknen entfernt. Als Bindemittel in der Dispersion kann beispielsweise Hydroxypropylcellulose, Hydroxypropylmethylcellulose, Methylcellulose, Hydroxyethylcellulose, Carboxymethylcellulose, Polyvinylpyrrolidan, Copolymerisate aus N-Vinylpyrrolidon und Vinylacetat oder Kombinationen dieser Polymere verwendet werden. Vorzugsweise wird Hydroxypropylcellulose oder Copolymerisate aus *N*-Vinylpyrrolidon und Vinylacetat eingesetzt. Als Trennmittel eignen sich z.B. Talkum, Magnesiumstarat oder Kieselsäure; vorzugsweise wird Talkum verwendet. Als Dispersionsmittel kommen beispielsweise Wasser, Ethanol, 2-Propanol, Aceton oder Mischungen dieser Lösungsmittel miteinander in Frage. Der Wirkstoffauftrag auf das Kernmaterial kann mittels in der pharmazeutischen Technologie bekannter und etablierter Verfahren z.B. in Dragierkesseln, konventionellen Filmcoatinganlagen oder in der Wirbelschicht erfolgen. Anschließend kann erneut ein Siebprozess durchgeführt werden.

Zur Verminderung eines erhöhten Abriebs bei der Abfüllung in Kapseln oder zur Erhöhung der Lagerstabilität kann das System abschließend noch mit einer Schicht aus pharmazeutisch üblichen Filmbildnern, Weichmachern und ggf. Pigmenten überzogen werden. Dies kann mit Hilfe von üblichen Verfahren erfolgen, wie sie bereits bei der Beschreibung des Aufbringens der Isolierschicht erwähnt wurden.

Bei Verwendung von Kernmaterial mit einem mittleren Durchmesser von 0,4 - 1,5 mm werden durch das oben beschriebene Verfahren wirkstoffhaltige Pellets erhalten, die anschließend beispielsweise in Hartkapseln abgefüllt werden können. Dazu wird eine der Dosierung entsprechende Vielzahl dieser Einheiten auf Standard-Kapselfüllmaschinen in Hartkapseln gefüllt. Als geeignete Hartkapseln kommen beispielsweise Hartgelatinekapseln oder Hartkapseln aus Hydroxypropylmethylcellulose (HPMC) in Frage. Der bevorzugte Wirkstoffgehalt der pharmazeutischen Zusammensetzung beträgt maximal 60 %, vorzugsweise maximal 50 %.

Soweit nicht anders vermerkt, handelt es sich bei den Prozentangaben stets um Gewichtsprozente. Angaben zum Wirkstoffgehalt beziehen sich, soweit nicht anders angegeben, jeweils auf die Wirkstoffbase.

Wie bereits erwähnt weisen die erfindungsgemäßen pharmazeutischen Zusammensetzungen eine verbesserte Bioverfügbarkeit des enthaltenen Wirkstoffs auf. Beispielsweise wurden die pharmazeutischen Zusammensetzungen gemäß der Beispiele 1 und 2 auf ihre Bioverfügbarkeitsverbesserung hin getestet.

*Ergebnisse der klinischen Prüfungen*

Dazu wurde die gemäß Beispiel 1 hergestellte Formulierung an insgesamt 15 Probanden im Hinblick auf ihre Bioverfügbarkeit klinisch geprüft. In einem Behandlungsast erhielten die Probanden die Zusammensetzung nüchtern per os (= orale Gabe) ohne eine Vorbehandlung. In einem weiteren Behandlungsast wurden die gleichen Probanden vor per os - Applikation der Zusammensetzung zur Erhöhung des Magen-pH drei Tage lang mit 40 mg Pantoprazol b.i.d. (= zwei mal täglich) per os vorbehandelt; die Behandlung mit Pantoprazol wurde während der Verabreichung der Formulierung fortgeführt.

Das Ausmaß der Absorption wurde über die quantitative Bestimmung der Urinausscheidung des wirksamen Metaboliten 3-[(2-{[4-(Amino-imino-methyl)-phenylamino]-methyl}-1-methyl-1*H*-benzimidazol-5-carbonyl)-pyridin-2-yl-amino]-propionsäure ermittelt.

Die relative Bioverfügbarkeit nach Vorbehandlung mit Pantoprazol lag im Vergleich zur Applikation ohne vorhergehende Vorbehandlung im Mittel bei 94 %.

Unter vergleichbaren Applikationsbedingungen liegt die relative Bioverfügbarkeit (basierend auf der Fläche unter der Plasmakonzentrations-Zeit-Kurve) einer 50 mg Wirkstoff enthaltenden Tablette, die nach dem Stand der Technik entwickelt und hergestellt wurde und keine wasserlösliche organische Säure enthält, nach entsprechender Vorbehandlung mit Pantoprazol bei 18 %. Die folgende Liste gibt die genaue Zusammensetzung der verwendeten Tablette an:

Tabelle 1:

| | Bestandteil | mg/Tablette |
|---|---|---|
| **Kern** | 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester Mesylat | 57.7 |
| | Lactose monohydrat | 58.0 |
| | Microkristalline Cellulose | 48.3 |
| | Crospovidon | 3.4 |
| | Magnesiumstearat | 2.6 |
| **Filmüberzug** | Polyethylenglycol 6000 | 0.56 |
| | Titandioxid | 0.80 |
| | Talkum | 0.64 |
| | Hydroxypropylmethylcellulose | 1.92 |
| | Eisenoxid gelb | 0.08 |
| | Total | 174.0 |

Die Bioverfügbarkeit wurde durch Verwendung der erfindungsgemäßen Formulierung also ca. um den Faktor 5 verbessert.

Die gemäß Beispiel 2 hergestellte Formulierung wurde ebenfalls an insgesamt 15 Probanden im Hinblick auf ihre Bioverfügbarkeit klinisch geprüft. In einem Behandlungsast erhielten die Probanden die Zusammensetzung nüchtern per os ohne eine Vorbehandlung. In einem weiteren Behandlungsast wurden die gleichen Probanden vor per os - Applikation der Zusammensetzung zur Erhöhung des Magen-pH drei Tage lang mit 40 mg Pantoprazol b.i.d. per os vorbehandelt; die Behandlung wurde während der Verabreichung der erfindungsgemäßen Formulierung fortgeführt.

Das Ausmaß der Absorption wurde über die quantitative Bestimmung der Urinausscheidung des wirksamen Metaboliten 3-[(2-{[4-(Amino-imino-methyl)-phenyl-amino]-methyl}-1-methyl-1*H*-benzimidazol-5-carbonyl)-pyridin-2-yl-amino]-propionsäure ermittelt.

Die relative Bioverfügbarkeit nach Vorbehandlung mit Pantoprazol lag im Vergleich zur Applikation ohne vorhergehende Vorbehandlung im Mittel bei 76 %.

Unter vergleichbaren Applikationsbedingungen liegt die relative Bioverfügbarkeit (basierend auf der Fläche unter der Plasmakonzentrations-Zeit-Kurve) einer 50 mg Wirkstoff enthaltenden Tablette, die nach dem Stand der Technik entwickelt und hergestellt wurde und keine wasserlösliche organische Säure enthält, nach entsprechender Vorbehandlung mit Pantoprazol bei 18 %. Die folgende Liste gibt die genaue Zusammensetzung der verwendeten Tablette an:

Tabelle 2:

| | Bestandteil | mg/Tablette |
|---|---|---|
| Kern | 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester Mesylat | 57.7 |
| | Lactose monohydrat | 58.0 |
| | Microkristalline Cellulose | 48.3 |
| | Crospovidon | 3.4 |
| | Magnesiumstearat | 2.6 |
| Filmüberzug | Polyethylenglycol 6000 | 0.56 |
| | Titandioxid | 0.80 |
| | Talkum | 0.64 |
| | Hydroxypropylmethylcellulose | 1.92 |
| | Eisenoxid gelb | 0.08 |
| | Total | 174.0 |

Durch die Verwendung der erfindungsgemäßen Formulierung wurde die Bioverfügbarkeit des Wirkstoffs gegenüber herkömmlicher Formulierungen also ca. um den Faktor 4 verbessert.

Figur 2 stellt die Bioverfügbarkeit der erfindungsgemäßen Formulierungen im Vergleich zu der konventionellen Tablette mit bzw. ohne Gabe von Pantoprazol graphisch dar.

Die Menge an Wirkstoff pro Kapsel wird vorzugsweise so gewählt, daß zur Erzielung der gewünschten Wirkung die Einnahme von 1 bis 2 Kapseln täglich ausreicht.

Das bevorzugte Säure-Wirkstoffverhältnis beträgt ca. 1:1 bis ca. 20:1. Die theoretische Untergrenze, bei der das System noch funktionieren kann, liegt bei 1 Equivalent Säure pro Mol des Wirkstoffs. Die Obergrenze von ca. 20:1 (Säure zu Wirkstoff), wird durch die Größe der Darreichungsform bei den gewünschten Dosierungen bestimmt (Anzahl der Pellets pro Kapsel).

In der Qualitätskontrolle werden in vitro Freigaben mit USP Methoden gemessen. Hierbei wird die Arzneiform in einem Volumen von 900 ml freigesetzt und der pH so gewählt, daß "Sink-Conditions" bestehen, d.h. die vollständige Wirkstoffdosis ist in diesen 900 ml löslich. Diese in vitro Methodik kann nicht prädiktiv für die Resorption beim Menschen sein, da dieser Arzneimittel meist mit ca. 200 ml Flüssigkeit einnimmt und bei anazidem Magen die Löslichkeit in vielen Fällen nur für einen Bruchteil der Dosis ausreicht. Anazider Magen kommt bei älteren Patienten in einer Häufigkeit von ca 25 % der Bevölkerung vor, häufig wird er auch durch Comedikation mit H2-Blockern oder Protonenpumpeninhibitoren hervorgerufen.

Es wurde daher im Rahmen der Erfindung eine empirische Testmethodik entwickelt, die eine bessere Korrelation zur in vivo Performance am Mensch aufweist, insbesondere bei anazidem Magen. Hierzu wird eine Arzneiform, die die höchste am Menschen angewendete Dosis enthält, in einem Volumen von 200 ml (dies entspricht der Humananwendung) in Puffer bei einem pH Wert mit niedriger Löslichkeit des Wirkstoffs im physiologisch sinnvollen Bereich, d.h. zwischen pH 1 - 7 freigesetzt. Da sich mit dieser Methode die Resorbierbarkeit auch bei anazidem Magen-pH gut vorhersagen läßt, ist sie für die Optimierung von Arzneiformen gut geeignet. Um aus vielen Rezepturbeispielen die jeweils günstigste Formulierung zu identifizieren, kann die maximale Freigabe und/oder die Fläche unter dem Kurvenverlauf vom Zeitpunkt 0 bis zum Endpunkt der Freigabe als relevante Kenngrößen verwendet werden.

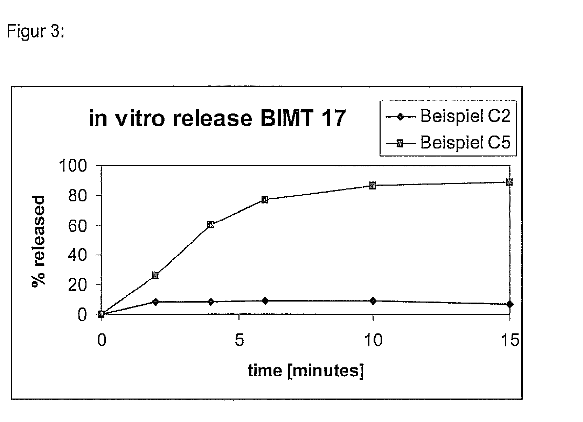

Dies wird am Beispiel des Vergleichs der Formulierungsbeispiele C2 (Referenz ohne Säurestarter) und C5 (erfindungsgemäßes Beispiel mit Weinsäurestarter) deutlich (Figur 3 und Tabelle 3).

Tabelle 3 zeigt die in vitro Freigaben und Kenndaten AUC und maximale Freigabe eines erfindungsgemäßen Beispiels C5 (Wirkstoff: Flibanserin; vgl. Tabelle 11) im Vergleich zur Referenzform mit ähnlichem Wirkstoffgehalt in 0.005 Mol Citratpuffer pH 5.0

Tabelle 3:

| Zeit [Minuten] | 0 | 2 | 4 | 6 | 10 | 15 | AUC 0-15' | max Freisetzung | pH* |
|---|---|---|---|---|---|---|---|---|---|
| Beispiel C2 | 0 | 8 | 8 | 9 | 9 | 7 | 115 | 9 | 4,90 |
| Beispiel C5 | 0 | 26 | 61 | 77 | 87 | 89 | 1018 | 89 | 3,45 |
| *: Dieser Wert stellt den pH Wert am Ende der Freigabe dar. | | | | | | | | | |

Tabelle 4 zeigt die in vitro Freigaben und Kenndaten AUC und maximale Freigabe der erfindungsgemäßen Beispiele C4 bis C15 (Wirkstoff: Flibanserin; vgl. Tabelle 11) im Vergleich zu Referenzformen C1 bis C3.

Tabelle 4:

| Zeit [Minuten] | 0 | 2 | 4 | 6 | 10 | 15 | AUC 0-15' | max Freisetzung | pH* |
|---|---|---|---|---|---|---|---|---|---|
| Beispiel C1 | 0 | 8 | 9 | 9 | 9 | 8 | 119 | 9 | 4,90 |
| Beispiel C2 | 0 | 8 | 8 | 9 | 9 | 7 | 115 | 9 | 4,90 |
| Beispiel C3 | 0 | 5 | 7 | 8 | 8 | 6 | 97 | 8 | 4,29 |
| Beispiel C4 | 0 | 65 | 91 | 95 | 97 | 101 | 1309 | 103 | 2,80 |
| Beispiel C5 | 0 | 26 | 61 | 77 | 87 | 89 | 1018 | 89 | 3,45 |
| Beispiel C6 | 0 | 11 | 19 | 29 | 45 | 50 | 476 | 50 | 3,95 |
| Beispiel C7 | 0 | 13 | 34 | 53 | 80 | 99 | 884 | 107 | 3,00 |
| Beispiel C8 | 0 | 5 | 11 | 22 | 55 | 100 | 598 | 100 | 4,00 |
| Beispiel C10 | 0 | 51 | 79 | 86 | 94 | 97 | 1182 | 97 | 3,98 |
| Beispiel C11 | 0 | 7 | 16 | 27 | 33 | 40 | 371 | 40 | 3,95 |
| Beispiel C14 | 0 | 26 | 77 | 88 | 91 | 93 | 1113 | 93 | 3,35 |
| Beispiel C15 | 0 | 16 | 36 | 37 | 36 | 37 | 471 | 37 | 3,95 |
| *: Dieser Wert stellt den pH Wert am Ende der Freigabe dar. | | | | | | | | | |

<u>Interpretation der Ergebnisse:</u>

Alle erfindungsgemäßen Beispiele sind der Referenzformulierung deutlich überlegen. Mit zunehmendem Wirkstoffgehalt nimmt die in vitro Freigabe etwas ab, da bei gleicher Wirkstoffdosis die Säuremenge geringer wird.

Alle Säuren und sonstigen Hilfsstoffe sind generell geeignet, zeigen aber bei vergleichbaren Wirkstoffkonzentration etwas unterschiedliches Freigabeverhalten. Als besonders geeignet im Sinne der Erfindung erwiesen sich die Beispiele C4, C5, C7, C8, C10 und C14.

Tabelle 5 zeigt die in vitro Freigaben und Kenndaten AUC und maximale Freigabe der erfindungsgemäßen Beispiele C18 bis C31 (Wirkstoff: Pimobendan; vgl. Tabelle 12) im Vergleich zu Referenzformen C16 bis C17. Als Testmedium diente ein 0.005 M Citratpuffer eingestellt auf pH 5.0.

Tabelle 5:

| Zeit [Minuten] | 0 | 2 | 4 | 6 | 10 | 15 | Max | AUC 1 bis 15 | pH* |
|---|---|---|---|---|---|---|---|---|---|
| Beispiel C16 | 0 | 11 | 11 | 15 | 15 | 14 | 16 | 194 | 5,06 |
| Beispiel C17 | 0 | 15 | 22 | 25 | 26 | 19 | 26 | 312 | 4,85 |
| Beispiel C18 | 0 | 53 | 61 | 74 | 87 | 91 | 91 | 1072 | 3,05 |
| Beispiel C19 | 0 | 35 | 52 | 59 | 68 | 71 | 71 | 834 | 3,2 |
| Beispiel C20 | 0 | 26 | 32 | 33 | 42 | 48 | 48 | 523 | 3,55 |
| Beispiel C21 | 0 | 31 | 51 | 57 | 62 | 66 | 66 | 781 | 3,5 |
| Beispiel C22 | 0 | 10 | 15 | 19 | 22 | 24 | 24 | 266 | 4,1 |
| Beispiel C23 | 0 | 6 | 10 | 13 | 15 | 16 | 16 | 179 | 4,2 |
| Beispiel C24 | 0 | 38 | 46 | 54 | 62 | 80 | 80 | 808 | 2,8 |
| Beispiel C25 | 0 | 13 | 18 | 26 | 44 | 59 | 59 | 485 | 3,55 |

(fortgesetzt)

| Zeit [Minuten] | 0 | 2 | 4 | 6 | 10 | 15 | Max | AUC 1 bis 15 | pH* |
|---|---|---|---|---|---|---|---|---|---|
| Beispiel C26 | 0 | 10 | 21 | 25 | 30 | 38 | 38 | 366 | 3,7 |
| Beispiel C27 | 0 | 52 | 73 | 79 | 85 | 85 | 86 | 1079 | 2,85 |
| Beispiel C28 | 0 | 19 | 47 | 56 | 64 | 71 | 71 | 765 | 3,3 |
| Beispiel C29 | 0 | 7 | 19 | 22 | 25 | 26 | 26 | 298 | 3,5 |
| Beispiel C30 | 0 | 50 | 70 | 78 | 84 | 87 | 87 | 1071 | 2,95 |
| Beispiel C31 | 0 | 27 | 42 | 47 | 52 | 54 | 54 | 648 | 3,6 |
| *: Dieser Wert stellt den pH Wert am Ende der Freigabe dar. | | | | | | | | | |

Interpretation der Ergebnisse:

Alle erfindungsgemäßen Beispiele sind der Referenzformulierung deutlich überlegen. Mit zunehmendem Wirkstoffgehalt nimmt die in vitro Freigabe etwas ab, da bei gleicher Wirkstoffdosis die Säuremenge geringer wird.

Alle Säuren und sonstigen Hilfsstoffe sind generell geeignet, zeigen aber bei vergleichbaren Wirkstoffkonzentration etwas unterschiedliches Freigabeverhalten. Als besonders geeignet im Sinne der Erfindung erwiesen sich die Beispiele C18, C19, C21, C24, C27, C28, C30 und C31.

Tabelle 6 zeigt die in vitro Freigaben und Kenndaten AUC und maximale Freigabe der erfindungsgemäßen Beispiele C35 bis C52 (Wirkstoff: Lefradafiban; vgl. Tabelle 13) im Vergleich zu Referenzformen C32 bis C34. Als Testmedium diente ein 0.005 M Citratpuffer eingestellt auf pH 5.0.

Tabelle 6:

| Zeit [Minuten] | 0 | 2 | 4 | 6 | 10 | 15 | Max | AUC 1 bis 15 | pH* |
|---|---|---|---|---|---|---|---|---|---|
| Beispiel C32 | 0 | 13 | 18 | 15 | 19 | 13 | 21 | 225 | 4,95 |
| Beispiel C33 | 0 | 10 | 13 | 18 | 17 | 18 | 20 | 218 | 4,90 |
| Beispiel C34 | 0 | 5 | 6 | 9 | 10 | 10 | 11 | 119 | 4,95 |
| Beispiel C35 | 0 | 102 | 106 | 101 | 100 | 101 | 106 | 1418 | 2,85 |
| Beispiel C36 | 0 | 49 | 75 | 86 | 95 | 94 | 95 | 1166 | 3,35 |
| Beispiel C37 | 0 | 8 | 20 | 33 | 33 | 37 | 37 | 395 | 4,00 |
| Beispiel C38 | 0 | 62 | 77 | 78 | 79 | 83 | 83 | 1076 | 3,50 |
| Beispiel C39 | 0 | 16 | 34 | 41 | 41 | 38 | 42 | 498 | 4,00 |
| Beispiel C40 | 0 | 7 | 10 | 15 | 23 | 24 | 24 | 245 | 4,30 |
| Beispiel C41 | 0 | 61 | 94 | 98 | 100 | 105 | 105 | 1318 | 2,70 |
| Beispiel C42 | 0 | 11 | 26 | 33 | 60 | 74 | 74 | 625 | 3,50 |
| Beispiel C43 | 0 | 7 | 10 | 16 | 29 | 44 | 44 | 322 | 3,90 |
| Beispiel C44 | 0 | 97 | 102 | 99 | 100 | 95 | 102 | 1387 | 2,60 |
| Beispiel C45 | 0 | 6 | 39 | 66 | 79 | 87 | 87 | 862 | 3,40 |
| Beispiel C46 | 0 | 4 | 20 | 27 | 33 | 34 | 34 | 367 | 3,95 |
| Beispiel C47 | 0 | 98 | 98 | 101 | 95 | 99 | 102 | 1372 | 2,75 |
| Beispiel C48 | 0 | 40 | 62 | 69 | 69 | 72 | 72 | 904 | 3,60 |
| Beispiel C49 | 0 | 4 | 13 | 27 | 32 | 32 | 32 | 341 | 3,95 |
| Beispiel C50 | 0 | 95 | 100 | 101 | 105 | 104 | 106 | 1426 | 2,85 |
| Beispiel C51 | 0 | 65 | 59 | 66 | 65 | 67 | 68 | 909 | 3,70 |
| Beispiel C52 | 0 | 36 | 37 | 39 | 41 | 41 | 42 | 549 | 4,05 |
| *: Dieser Wert stellt den pH Wert am Ende der Freigabe dar. | | | | | | | | | |

Interpretation der Ergebnisse:

Alle erfindungsgemäßen Beispiele sind der Referenzformulierung deutlich überlegen. Mit zunehmendem Wirkstoffgehalt nimmt die in vitro Freigabe etwas ab, da bei gleicher Wirkstoffdosis die Säuremenge geringer wird.

Alle Säuren und sonstigen Hilfsstoffe sind generell geeignet, zeigen aber bei vergleichbaren Wirkstoffkonzentration etwas unterschiedliches Freigabeverhalten.

Als besonders geeignet im Sinne der Erfindung erwiesen sich die Beispiele C35, C36, C38, C41, C42, C44, C45, C47, C48, C50, C51 und C52.

Tabelle 7 zeigt die in vitro Freigaben und Kenndaten AUC und maximale Freigabe der erfindungsgemäßen Beispiele C54 bis C59 (Wirkstoff: Amelubant; vgl. Tabelle 14) im Vergleich zur Referenzform C53. Als Testmedium diente aufgrund der sehr niedrigen Löslichkeit des Wirkstoffs eine Mischung aus Wasser/Äthanol 2/1.

Tabelle 7:

| Time [Min] | 0 | 5 | 10 | 15 | 20 | 25 | 30 | Max | AUC 1bis 3C | pH* |
|---|---|---|---|---|---|---|---|---|---|---|
| Beispiel C53 | 0 | 1 | 1 | 1 | 2 | 1 | 3 | 3 | 40 | 7,46 |
| Beispiel C54 | 0 | 53 | 54 | 55 | 58 | 57 | 62 | 62 | 1546 | 2,46 |
| Beispiel C55 | 0 | 34 | 39 | 39 | 41 | 43 | 44 | 44 | 1086 | 2,63 |
| Beispiel C56 | 0 | 12 | 12 | 12 | 12 | 12 | 11 | 13 | 335 | 3,47 |
| Beispiel C57 | 0 | 31 | 49 | 57 | 60 | 68 | 64 | 68 | 1484 | 2,46 |
| *: Dieser Wert stellt den pH Wert am Ende der Freigabe dar. | | | | | | | | | | |

Interpretation der Ergebnisse:

Alle erfindungsgemäßen Beispiele sind der Referenzformulierung deutlich überlegen, wobei in diesem Fall Bernsteinsäurestarterpellets schlechter abschneiden als Wein- und Fumarsäure.

Als besonders geeignet im Sinne der Erfindung erwiesen sich die Beispiele C54 bis C57.

Tabelle 8 zeigt die in vitro Freigaben und Kenndaten AUC und maximale Freigabe der erfindungsgemäßen Beispiele C60 bis C61 (Wirkstoff: Telmisartan; vgl. Tabelle 15) im Vergleich zu Referenzformen C58 und C59. Als Testmedium diente ein 0.0005 M Citratpuffer eingestellt auf pH 5.0.

Tabelle 8:

| Time [min] | 0 | 2 | 4 | 6 | 10 | 15 Max | | AUC bis 30 pH* | |
|---|---|---|---|---|---|---|---|---|---|
| Beispiel C58 | 0 | 0 | 1 | 2 | 2 | 2 | 2 | 20,64 | 5 |
| Beispiel C59 | 0 | 0 | 0 | 0 | 0 | 0 | 0 | 4,57 | 5 |
| Beispiel C60 | 0 | 15 | 20 | 25 | 26 | 24 | 26 | 322,11 | 2 |
| Beispiel C61 | 0 | 5 | 6 | 7 | 7 | 8 | 8 | 93,11 | 2 |
| *: Dieser Wert stellt den pH Wert am Ende der Freigabe dar. | | | | | | | | | |

Interpretation der Ergebnisse:

Alle erfindungsgemäßen Beispiele sind der Referenzformulierung deutlich überlegen.

Mit zunehmendem Wirkstoffgehalt nimmt die in vitro Freigabe ab, da bei gleicher Wirkstoffdosis die Säuremenge geringer wird.

Als besonders geeignet im Sinne der Erfindung erwiesen sich die Beispiele C60 und C61.

Legende zu den Figuren:

Figur 1 zeigt den schematischen Aufbau einer erfindungsgemäßen pharmazeutischen Zusammensetzung in Form einer Schnittdarstellung eines Pellets.

Figur 2 stellt die Bioverfügbarkeit der erfindungsgemäßen Formulierungen im Vergleich zur konventionellen Tablette mit bzw. ohne Gabe von Pantoprazol graphisch dar.

Figur 3 zeigt die in vitro Freigaben eines erfindungsgemäßen Beispiels C5 im Vergleich zur Referenzformulierung C2 mit ähnlichem Wirkstoffgehalt in 0.005 Mol Citratpuffer pH 5.0.

Die nachfolgenden Beispiele sollen die Erfindung näher erläutern:

**Beispiele:**

*Beispiel 1*

Tabelle 9:

| | Prozentuale Zusammensetzung | | | | pro Kapsel |
|---|---|---|---|---|---|
| | Kernmaterial | Isolierschicht | Wirkstoff schicht | Gesamt | [mg] |
| Weinsäure | 61,3 | - | - | 61,3 | 176,7 |
| Gummi arabicum | 3,1 | 2,8 | | 5,9 | 17,0 |
| Talkum | - | 5,6 | 3,2 | 8,8 | 25,4 |
| Hydroxypropylcellulose | - | - | 4,0 | 4,0 | 11,5 |
| Wirkstoff (3-[(2-{[4-(Hexyloxycarbonylamino-imino-methyl)-phenyl-amino]-methyl}-1-methyl-1*H*-benzimidazol-5-carbonyl)-pyridin-2-yl-amino]-propionsäure-ethylester Mesylat) | - | - | 20,0 | 20,0 | 57,7* |
| Summe | | | | 100,0 | 288,3 |
| *) entspricht 50 mg 3-[(2-{[4-(Hexyloxycarbonylamino-imino-methyl)-phenylamino]-methyl}-1-methyl-1*H*-benzimidaol-5-carbonyl)-pyridir-2-yl-amino]-propionsäure-ethylester (Wirkstoffbase) | | | | | |

*a) Aufbau von weinsäurehaltigem Kernmaterial*

<u>Zusammensetzung:</u>

Gummi arabicum    1 Gewichtsteil
Weinsäure    20 Gewichtsteile

In 4 Gewichtsteilen gereinigtem Wasser von 50°C wird unter Rühren 1 Gewichtsteil Gummi arabicum gelöst. In dieser Lösung werden anschließend unter Rühren 5 Gewichtsteile Weinsäure gelöst.

In einer geeigneten, mit einer Zu- und Ablufteinrichtung versehenen Dragieranlage werden 8,3 Gewichtsteile Weinsäurekristalle mit einer mittleren Partikelgröße von 0,4 bis 0,6 mm vorgelegt und der Kessel in Rotation versetzt. Bei einer Zulufttemperatur von 60° - 80° C werden die Weinsäurekristalle im Intervallbetrieb mit der Weinsäure-Gummi arabicum - Lösung besprüht und mit insgesamt 6,7 Gewichtsteilen pulverförmiger Weinsäure bepudert, so dass annähernd sphärische Partikel entstehen.

Das sphärische Weinsäurekernmaterial wird anschließend im rotierenden Kessel bei einer Zulufttemperatur von 60° - 80° C nachgetrocknet.

Das Kernmaterial wird über eine Taumelsiebmaschine mit Siebböden mit einer nominalen Maschenweite von 0,6 und 0,8 mm fraktioniert. Die Gutfraktion zwischen 0,6 und 0,8 mm wird im weiteren Prozess verwendet.

*b) Isolierung des weinsäurehaltigen Kernmaterials*

<u>Zusammensetzung:</u>

Weinsäurehaltiges Kernmaterial    23 Gewichtsteile
Gummi arabicum    1 Gewichtsteil
Talkum    2 Gewichtsteile

In einer Mischung aus 6,7 Gewichtsteilen Ethanol 96 % und 13,5 Gewichtsteilen gereinigtem Wasser wird unter Rühren 1 Gewichtsanteil Gummi arabicum gelöst. Anschließend werden in der Lösung unter Rühren 2 Gewichtsanteile Talkum dispergiert.

In einer Wirbelschichtprozessanlage werden 23 Gewichtanteile weinsäurehaltiges Kernmaterial bei einer Zulufttemperatur von 35° - 40° C mit der Gummi arabicum-Talkum - Dispersion im Unterbettsprühverfahren besprüht.

Das isolierte weinsäurehaltige Kernmaterial wird anschließend im Umlufttrockenschrank bei 40° C über 8 Stunden nachgetrocknet.

Zur Entfernung von Agglomeraten wird das getrocknete isolierte weinsäurehaltige Kernmaterial mit einem Sieb mit der nominalen Maschenweite 1,0 mm gesiebt. Die Gutfraktion (Korngröße < 1 mm) wird weiterverarbeitet.

c) Aufbau der Wirkstoffschicht

|  Zusammensetzung: | |
| --- | --- |
| isoliertes weinsäurehaltiges Kernmaterial | 91 Gewichtsteile |
| Hydroxypropylcellulose | 5 Gewichtsteile |
| Talkum | 4 Gewichtsteile |
| Wirkstoff (Mesylat von BIBR 1048) | 25 Gewichtsteile |

In 168 Gewichtsteilen 2-Propanol wird Hydroxypropylcellulose unter Rühren gelöst und anschließend in dieser Lösung der Wirkstoff und Talkum unter Rühren dispergiert.

In einer Wirbelschichtprozessanlage werden 91 Gewichtanteile isoliertes weinsäurehaltiges Kernmaterial bei einer Zulufttemperatur von 20° - 30° C mit der wirkstoffhaltigen Dispersion im Unterbettsprühverfahren besprüht.

Die wirkstoffhaltigen Pellets werden anschließend im Umlufttrockenschrank bei 35° C über 8 Stunden nachgetrocknet.

Zur Entfernung von Agglomeraten werden die wirkstoffhaltigen Pellets mit einem Sieb mit der nominalen Maschenweite 1,25 mm gesiebt. Die Gutfraktion (Korngröße < 1,25 mm) wird weiterverarbeitet.

d) Abfüllung in Kapseln

Eine jeweis 50 mg Wirkstoffbase enthaltende Menge an wirkstoffhaltigen Pellets wird mittels einer Kapselfüllmaschine in Hartgelatinekapseln der Größe 1 abgefüllt.

Beispiel 2

Tabelle 10:

| | Prozentuale Zusammensetzung | | | | pro Kapsel |
| --- | --- | --- | --- | --- | --- |
| | Kernmaterial | Isolierschicht | Wirkstoff schicht | Gesamt | [mg] |
| Weinsäure | 38,5 | - | - | 38,5 | 55,5 |
| Gummi arabicum | 1,9 | 1,7 | | 3,6 | 5,2 |
| Talkum | - | 3,5 | 6,4 | 9,9 | 14,3 |
| Hydroxypropylcellulose | - | - | 8,0 | 8,0 | 11,5 |
| Wirkstoff (3-[(2-{[4-(Hexyloxycarbonylamino-imino-methyl)-phenyl-amino]-methyl}-1-methyl-1H-benzimidazol-5-carbonyl)-pyridin-2-yl-amino]-propionsäure-ethylester Mesylat) | - | - | 40,0 | 40,0 | 57,7* |

(fortgesetzt)

| | Prozentuale Zusammensetzung | | | | pro Kapsel |
|---|---|---|---|---|---|
| | Kernmaterial | Isolierschicht | Wirkstoff schicht | Gesamt | [mg] |
| Summe | | | | 100,0 | 144,1 |

*) entspricht 50 mg 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester (Wirkstoffbase)

*a) Aufbau von weinsäurehaltigem Kernmaterial*

Zusammensetzung:
Gummi arabicum        1 Gewichtsteil
Weinsäure             20 Gewichtsteile

In 4 Gewichtsteilen gereinigtem Wasser von 50°C wird unter Rühren 1 Gewichtsteil Gummi arabicum gelöst. In dieser Lösung werden anschließend unter Rühren 5 Gewichtsteile Weinsäure gelöst.

In einer geeigneten, mit einer Zu- und Ablufteinrichtung versehenen Dragieranlage werden 8,3 Gewichtsteile Weinsäurekristalle mit einer mittleren Partikelgröße von 0,4 bis 0,6 mm vorgelegt und der Kessel in Rotation versetzt. Bei einer Zulufttemperatur von 60° - 80° C werden die Weinsäurekristalle im Intervallbetrieb mit der Weinsäure-Gummi arabicum - Lösung besprüht und mit insgesamt 6,7 Gewichtsteilen pulverförmiger Weinsäure bepudert, so dass annähernd sphärische Partikel entstehen.

Das sphärische Weinsäurekernmaterial wird anschließend im rotierenden Kessel bei einer Zulufttemperatur von 60° - 80° C nachgetrocknet.

Das Kernmaterial wird über eine Taumelsiebmaschine mit Siebböden mit einer nominalen Maschenweite von 0,6 und 0,8 mm fraktioniert. Die Gutfraktion zwischen 0,6 und 0,8 mm wird im weiteren Prozess verwendet.

*b) Isolierung des weinsäurehaltigen Kernmaterials*

Zusammensetzung:
Weinsäurehaltiges Kernmaterial    23 Gewichtsteile
Gummi arabicum                     1 Gewichtsteil
Talkum                             2 Gewichtsteile

In einer Mischung aus 6,7 Gewichtsteilen Ethanol 96 % und 13,5 Gewichtsteilen gereinigtem Wasser wird unter Rühren 1 Gewichtsanteil Gummi arabicum gelöst. Anschließend werden in der Lösung unter Rühren 2 Gewichtsanteile Talkum dispergiert.

In einer Wirbelschichtprozessanlage werden 23 Gewichtanteile weinsäurehaltiges Kernmaterial bei einer Zulufttemperatur von 35° - 40° C mit der Gummi arabicum-Talkum - Dispersion im Unterbettsprühverfahren besprüht.

Das isolierte weinsäurehaltige Kernmaterial wird anschließend im Umlufttrockenschrank bei 40° C über 8 Stunden nachgetrocknet.

Zur Entfernung von Agglomeraten wird das getrocknete isolierte weinsäurehaltige Kernmaterial mit einem Sieb mit der nominalen Maschenweite 1,0 mm gesiebt. Die Gutfraktion (Korngröße < 1 mm) wird weiterverarbeitet.

*c) Aufbau der Wirkstoffschicht*

Zusammensetzung:
Isoliertes weinsäurehaltiges Kernmaterial    57 Gewichtsteile
Hydroxypropylcellulose                       10 Gewichtsteile

(fortgesetzt)

| Zusammensetzung: | |
| --- | --- |
| Talkum | 8 Gewichtsteile |
| Wirkstoff (Mesylat von BIBR 1048) | 50 Gewichtsteile |

In 335 Gewichtsteilen 2-Propanol wird Hydroxypropylcellulose unter Rühren gelöst und anschließend in dieser Lösung der Wirkstoff und Talkum unter Rühren dispergiert.

In einer Wirbelschichtprozessanlage werden 91 Gewichtanteile isoliertes weinsäurehaltiges Kernmaterial bei einer Zulufttemperatur von 20° - 30° C mit der wirkstoffhaltigen Dispersion im Unterbettsprühverfahren besprüht.

Die wirkstoffhaltigen Pellets werden anschließend im Umlufttrockenschrank bei 35° C über 8 Stunden nachgetrocknet.

Zur Entfernung von Agglomeraten werden die wirkstoffhaltigen Pellets mit einem Sieb mit der nominalen Maschenweite 1,25 mm gesiebt. Die Gutfraktion (Korngröße < 1,25 mm) wird weiterverarbeitet.

*d) Abfüllung in Kapseln*

Eine jeweis 50 mg Wirkstoffbase enthaltende Menge an wirkstoffhaltigen Pellets wird mittels einer Kapselfüllmaschine in Hartgelatinekapseln der Größe 2 abgefüllt.

*Weitere Beispiele*

Die Herstellung der nachfolgenden Beispiele erfolgt generell in 5 Arbeitsschritten:

a: *Aufbau von säurehaltigem Kernmaterial*
b: *Isolierung des säurehaltigen Kernmaterials*
c: *Aufbau der Wirkstoffschicht*
d: I*solierung des Pellets bestehend aus säurehaltigem Kern*
e: *Abfüllung in Kapseln*

Schritt b ist bei Inkompatibilität zwischen Säure und Wirkstoffschicht zwingend notwendig, Schritt d ist notwendig, wenn die mechanische Stabilität der Wirkstoffschicht nicht für vollständige Auflösung des Wirkstoffs ausreicht.

a: *Beispiele für den Aufbau von säurehaltigem Kernmaterial*

Beispiel a1: Aufbau von weinsäurehaltigem Kernmaterial

| Zusammensetzung: | |
| --- | --- |
| Gummi arabicum | 1 Gewichtsteil |
| Weinsäure | 20 Gewichtsteile |

In 4 Gewichtsteilen gereinigtem Wasser von 50°C wird unter Rühren 1 Gewichtsteil Gummi arabicum gelöst. In dieser Lösung werden anschließend unter Rühren 5 Gewichtsteile Weinsäure gelöst.

In einer geeigneten, mit einer Zu- und Ablufteinrichtung versehenen Dragieranlage werden 8,3 Gewichtsteile Weinsäurekristalle mit einer mittleren Partikelgröße von 0,4 bis 0,6 mm vorgelegt und der Kessel in Rotation versetzt. Bei einer Zulufttemperatur von 60° - 80° C werden die Weinsäurekristaile im Intervallbetrieb mit der Weinsäure-Gummi arabicum - Lösung besprüht und mit insgesamt 6,7 Gewichtsteilen pulverförmiger Weinsäure bepudert, so dass annähernd sphärische Partikel entstehen.

Das sphärische Weinsäurekernmaterial wird anschließend im rotierenden Kessel bei einer Zulufttemperatur von 60° - 80° C nachgetrocknet.

Das Kernmaterial wird über eine Taumelsiebmaschine mit Siebböden mit einer nominalen Maschenweite von 0,6 und 0,8 mm fraktioniert. Die Gutfraktion zwischen 0,6 und 0,8 mm wird im weiteren Prozess verwendet.

Beispiel a2: Aufbau von fumarsäurehaltigem Kernmaterial

Zusammensetzung:
| | |
|---|---|
| Kollidon K25 | 3 Gewichtsteile |
| Avicel PH101 | 20 Gewichtsteile |
| Fumarsäure | 77 Gewichtsteile |

77 Gewichtsteile Fumarsäure, 20 Gewichtsteile Avicel PH 101 und 3 Gewichtsteile Kollidon K25 werden in einem Rhönradmischer 15 Minuten lang gemischt. Dann wird die Pulvermischung in einen Doppelschnecken-Dosierer überführt. Diese Mischung wird mit einer Geschwindigkeit von ca 1kg/h zusammen mit Wasser, das mit einer ProMinent Dosierpumpezugegeben wird in einen DoppelschneckenExtruder Typ Werner & Pfleiderer 37/18D (oder einen anderen geeigneten Extrudertyp) eingebracht. Die Wasserdosierung wird automatisch so geregelt, daß im Extruder ein Solldrehmoment ca. 19% erzeugt wird. Die Extrusion erfolgt über eine Düsenplatte mit Bohrungen von 8 mm Durchmesser.
Die Ausrundung der Extrusionsstränge zu Pellets erfolgt in einem WyPro-Sphäromat, wobei ca. 3Min bei ca. 850 RPM gerundet wurde.
Trocknung der Pellets bei 80°C ca. 1,5h im GPCG1 Wirbelschichttrockner.
Das Kernmaterial wird über eine Taumelsiebmaschine mit unterschiedlichen Siebböden mit nominalen Maschenweite von 0,71 bis 1,25 mm fraktioniert. Die jeweils geeigneten Gutfraktion zwischen 0,71 und 0,90 bzw. 0,90 und 1,12 mm werden in den weiteren Prozessen verwendet.

Beispiel a3: Aufbau von bernsteinsäurehaltigem Kernmaterial

Zusammensetzung:
| | |
|---|---|
| Kollidon K25 | 3 Gewichtsteile |
| Avicel PH101 | 20 Gewichtsteile |
| Bernsteinsäure | 77 Gewichtsteile |

77 Gewichtsteile Bernsteinsäure, 20 Gewichtsteile Avicel PH 101 und 3 Gewichtsteile Kollidon K25 werden in einem Rhönradmischer 15 Minuten lang gemischt.
Dann wird die Pulvermischung in einen Zwangsmischer überführt. Zu dieser Mischung wird unter Rühren Wasser zugegeben, bis eine extrusionsfähige, homogene Masse entsteht. Diese Mischung wird dann analog zur Herstellung der fumarsäurehaltigen Starterkerne weiterverarbeitet.
Die jeweils geeigneten Gutfraktion zwischen 0,71 und 0,90 bzw 0,90 und 1,12 mm werden in den weiteren Prozessen verwendet.

Beispiel a4: Aufbau von zitronensäurehaltiqem Kernmaterial

Zusammensetzung:
| | |
|---|---|
| Kollidon K90 | 1 Gewichtsteil |
| Talkum | 1 Gewichtsteil |
| Zitronensäuresäure | 20 Gewichtsteile |

In 6 Gewichtsteilen Isopropanol wird unter Rühren 1 Gewichtsteil Kollidon K90 gelöst und 1 Gewichtsteil Talkum wird einsuspendiert. In weiteren 6 Gewichtsteilen Isopropanol werden unter Rühren 3 Gewichtsteile Zitronensäure gelöst. In einer Wirbelschichtprozessanlage werden 17 Gewichtanteile isoliertes zitronensäurehaltiges Kernmaterial der Größe 0.5 - 0.6 mm bei einer Zulufttemperatur von 20° - 30° C mit den beiden wirkstoffhaltigen Dispersionen/Lösungen mittels einer Dreikopfdüse im Unterbettsprühverfahren besprüht, so dass annähernd sphärische Partikel entstehen.
Die wirkstoffhaltigen Pellets werden anschließend im Umlufttrockenschrank bei 35° C über 8 Stunden nachgetrocknet.

Das sphärische Weinsäurekernmaterial wird anschließend im rotierenden Kessel bei einer Zulufttemperatur von 60° - 80° C nachgetrocknet.

Das Kernmaterial wird über eine Taumelsiebmaschine mit Siebböden mit einer nominalen Maschenweite von 0,6 und 0,8 mm fraktioniert. Die Gutfraktion zwischen 0,6 und 0,8 mm wird im weiteren Prozess verwendet.

*b: Beispiel für die Isolierung des säurehaltigen Kernmaterials*

| Zusammensetzung: | |
| --- | --- |
| Säurehaltiges Kernmaterial | 23 Gewichtsteile |
| Gummi arabicum | 1 Gewichtsteil |
| Talkum | 2 Gewichtsteile |

In einer Mischung aus 6,7 Gewichtsteilen Ethanol 96 % und 13,5 Gewichtsteilen gereinigtem Wasser wird unter Rühren 1 Gewichtsanteil Gummi arabicum gelöst. Anschließend werden in der Lösung unter Rühren 2 Gewichtsanteile Talkum dispergiert.

In einer Wirbelschichtprozessanlage werden 23 Gewichtanteile säurehaltiges Kernmaterial bei einer Zulufttemperatur von 35° - 40° C mit der Gummi arabicum-Talkum - Dispersion im Unterbettsprühverfahren besprüht.

Das isolierte weinsäurehaltige Kernmaterial wird anschließend im Umlufttrockenschrank bei 40° C über 8 Stunden nachgetrocknet.

Zur Entfernung von Agglomeraten wird das getrocknete isolierte weinsäurehaltige Kernmaterial mit einem Sieb mit der nominalen Maschenweite 1,0 mm gesiebt. Die Gutfraktion (Korngröße < 1 mm) wird weiterverarbeitet.

*c: Beispiele für den Aufbau der Wirkstoffschicht*

Der Aufbau der Wirkstoffschicht erfolgt generell in immer gleicher Weise, wobei aber Wirkstoffart und Menge, Bindemittelart und Menge, Talkummenge und Isopropanol bzw Äthanolmenge variiert werden. Die Herstellung wird deshalb nur für Beispiel C4 beschrieben, die jeweiligen Zusammensetzungen werden für jeden Wirkstoff in Tabellenform dargestellt.

Herstellung von Beispiel C4:

| Zusammensetzung: | |
| --- | --- |
| Isoliertes weinsäurehaltiges Kernmaterial | 74 Gewichtsteile |
| Hydroxypropylcellulose | 49 Gewichtsteile |
| Talkum | 12 Gewichtsteile |
| Wirkstoff (z.B. Flibanserin) | 25 Gewichtsteile |

In 492 Gewichtsteilen 2-Propanol wird Hydroxypropylcellulose unter Rühren gelöst und anschließend in dieser Lösung der Wirkstoff und Talkum unter Rühren dispergiert.

In einer Wirbelschichtprozessanlage werden 74 Gewichtanteile isoliertes weinsäurehaltiges Kernmaterial bei einer Zulufttemperatur von 20° - 30° C mit der wirkstoffhaltigen Dispersion im Unterbettsprühverfahren besprüht.

Die wirkstoffhaltigen Pellets werden anschließend im Umlufttrockenschrank bei 35° C über 8 Stunden nachgetrocknet.

Zur Entfernung von Agglomeraten werden die wirkstoffhaltigen Pellets mit einem Sieb mit der nominalen Maschenweite 1,25 mm gesiebt. Die Gutfraktion (Korngröße < 1,25 mm) wird weiterverarbeitet.

Tabelle 11 zeigt die Zusammensetzung der Beispiele C1 - C 15 (Wirkstoff Flibanserin). Es sind jeweils Gewichtsteile angegeben, die dem experimentell ermittelten Wirkstoffgehalt entsprechen, d.h. die Sprühverluste, die von Charge zu Charge etwas schwanken können, wurden jeweils in der Rechnung kompensiert, um wirklich vergleichbare Daten zu erhalten.

Die Beispiele C1 - C3 enthalten einen im Handel erhältlichen Neutralkern anstelle der erfindungsgemäßen säurehaltigen Starterkerne. Diese Kerne dienen als Referenzwerte für die in vitro Testung (siehe oben).

Tabelle 12 zeigt die Zusammensetzung der Beispiele C16 - C 31 (Wirkstoff Pimobendan). Es sind jeweils Gewichtsteile angegeben, die dem experimentell ermittelten Wirkstoffgehalt entsprechen, d.h. die Sprühverluste, die von Charge zu Charge etwas schwanken können, wurden jeweils in der Rechnung kompensiert, um wirklich vergleichbare Daten zu erhalten.

Die Beispiele C16 -C17 enthalten einen im Handel erhältlichen Neutralkern anstelle der erfindungsgemäßen säurehaltigen Starterkerne. Diese Kerne dienen als Referenzwerte für die in vitro Testung (siehe oben).

Tabelle 13 zeigt die Zusammensetzung der Beispiele C32 - C 52 (Wirkstoff Lefradafiban, BIBU 104). Es sind jeweils Gewichtsteile angegeben, die dem experimentell ermittelten Wirkstoffgehalt entsprechen, d.h. die Sprühverluste, die von Charge zu Charge etwas schwanken können, wurden jeweils in der Rechnung kompensiert, um wirklich vergleichbare Daten zu erhalten.

Die Beispiele C32 - C34 enthalten einen im Handel erhältlichen Neutralkern anstelle der erfindungsgemäßen säurehaltigen Starterkerne. Diese Kerne dienen als Referenzwerte für die in vitro Testung (siehe oben).

Tabelle 14 zeigt die Zusammensetzung der Beispiele C53 - C 57 (Wirkstoff BIIL 284 = Amelubant). Es sind jeweils Gewichtsteile angegeben, die dem experimentell ermittelten Wirkstoffgehalt entsprechen, d.h. die Sprühverluste, die von Charge zu Charge etwas schwanken können, wurden jeweils in der Rechnung kompensiert, um wirklich vergleichbare Daten zu erhalten.

Das Beispiel C53 enthält einen im Handel erhältlichen Neutralkern anstelle der erfindungsgemäßen säurehaltigen Starterkerne. Dieser Kerne dient als Referenzwerte für die in vitro Testung (siehe oben).

Tabelle 15 zeigt die Zusammensetzung der Beispiele C58 - C 61 (Wirkstoff Pimobendan). Es sind jeweils Gewichtsteile angegeben, die dem experimentell ermittelten Wirkstoffgehalt entsprechen, d.h. die Sprühverluste, die von Charge zu Charge etwas schwanken können, wurden jeweils in der Rechnung kompensiert, um wirklich vergleichbare Daten zu erhalten.

Die Beispiele C58 - C59 enthalten einen im Handel erhältlichen Neutralkern anstelle der erfindungsgemäßen säurehaltigen Starterkerne. Diese Kerne dienen als Referenzwerte für die in vitro Testung (siehe oben).

Tabelle 11:

| | Neutral-Pellets | Weinsäure-pellets | Bernsteinsäure-Pellets | Fumarsäure-Pellets | Flibanserin | Povidone K25 | Talkum | Klucel EF | Isopropanol (nur zur Herstellung) | Wirkstoff-gehalt [%] |
|---|---|---|---|---|---|---|---|---|---|---|
| Beispiel C1 | 200 | | | | 17 | | 8 | 4 | 268 | 6.9 |
| Beispiel C2 | 200 | | | | 76 | | 38 | 19 | 885 | 22.7 |
| Beispiel C3 | 200 | | | | 127 | | 64 | 32 | 1272 | 31.4 |
| Beispiel C4 | | 200 | | | 35 | | 17 | 9 | 348 | 12.6 |
| Beispiel C5 | | 200 | | | 89 | | 44 | 22 | 888 | 24.8 |
| Beispiel C6 | | 200 | | | 204 | | 102 | 51 | 2040 | 37.7 |
| Beispiel C7 | | | | 200 | 43 | | 22 | 11 | 432 | 14.9 |
| Beispiel C8 | | | | 200 | 96 | | 48 | 24 | 960 | 24.7 |
| Beispiel C9 | | | | 200 | 170 | | 85 | 43 | 1704 | 34.6 |
| Beispiel C10 | | | 200 | | 22 | | 11 | 5 | 216 | 9.2 |
| Beispielen C11 | | | 200 | | 84 | | 42 | 21 | 840 | 22.7 |
| Beispiel C12 | | | 200 | | 168 | | 84 | 42 | 1680 | 36.5 |
| Beispiel C13 | | | 200 | | 31 | 4 | 16 | | 488 | 11.9 |
| Beispiel C14 | | | 200 | | 84 | 11 | 42 | | 999 | 24.4 |
| Beispiel C15 | | | 200 | | 156 | 20 | 78 | | 1454 | 35.5 |

Tabelle 12:

| | Neutral-pellets | Weinsäure-Starter | Bernsteinsäure-Starter | Fumarsäure-starter | Pimobendan | Povidone K90 | Talkum | Klucel EF | Isopropanol (nur zur Herstellung) | Wirkstoff-gehalt [%] |
|---|---|---|---|---|---|---|---|---|---|---|
| Beispiel C16 | 200 | | | | 10 | 3 | 5 | | 14 | 5 |
| Beispiel C17 | 200 | | | | 28 | 9 | 14 | | 38 | 11 |
| Beispiel C18 | | 200 | | | 12 | 4 | 6 | | 16 | 5 |
| Beispiel C19 | | 200 | | | 30 | 10 | 15 | | 40 | 11 |
| Beispiel C20 | | 200 | | | 55 | 18 | 27 | | 73 | 18 |
| Beispiel C21 | | | 200 | | 13 | 4 | 6 | | 17 | 5 |
| Beispiel C22 | | | 200 | | 41 | 14 | 20 | | 54 | 14 |
| Beispiel C23 | | | 200 | | 62 | 21 | 31 | | 83 | 21 |
| Beispiel C24 | | | | 200 | 16 | 5 | 8 | | 21 | 7 |
| Beispiel C25 | | | | 200 | 30 | 10 | 15 | | 40 | 12 |
| Beispiel C26 | | | | 200 | 81 | 27 | 41 | | 108 | 24 |
| Beispiel C27 | | 200 | | | 10 | | 5 | 3 | 13 | 4 |
| Beispiel C28 | | 200 | | | 33 | | 17 | 11 | 46 | 12 |
| Beispiel C29 | | 200 | | | 45 | | 23 | 15 | 60 | 16 |
| Beispiel C30 | | 200 | | | 13 | 2 | 6 | 2 | 17 | 5 |
| Beispiel C31 | | 200 | | | 37 | 6 | 19 | 6 | 50 | 13 |

Tabelle 13:

| | Neutral-pellets | Zitronensäure-Starter | Weinsäure-Starter | Bernsteinsäure-Starter | Fumarsäure-starter | Lefradafiban | Povidone K90 | Talkum | Klucel EF | Isopropanol (nur zur Herstellung) | exp. Wirk-stoffgehalt [%] |
|---|---|---|---|---|---|---|---|---|---|---|---|
| Beispiel C32 | 200 | | | | | 28 | 9 | 14 | | 184 | 10,8 |
| Beispiel C33 | 200 | | | | | 94 | 31 | 47 | | 624 | 25,3 |
| Beispiel C34 | 200 | | | | | 163 | 54 | 82 | | 1088 | 33,3 |
| Beispiel C35 | | | 200 | | | 28 | 9 | 14 | | 184 | 10,9 |
| Beispiel C36 | | | 200 | | | 82 | 27 | 41 | | 544 | 23,1 |
| Beispiel C37 | | | 200 | | | 160 | 53 | 80 | | 1064 | 33,5 |
| Beispiel C38 | | | | 200 | | 24 | 8 | 12 | | 160 | 10,1 |
| Beispiel C39 | | | | 200 | | 82 | 27 | 41 | | 544 | 23,3 |
| Beispiel C40 | | | | 200 | | 163 | 54 | 82 | | 1088 | 32,7 |
| Beispiel C41 | | | | | 200 | 24 | 8 | 12 | | 160 | 9,8 |
| Beispiel C42 | | | | | 200 | 86 | 29 | 43 | | 576 | 23,6 |
| Beispiel C43 | | | | | 200 | 94 | 31 | 47 | | 624 | 32,1 |
| Beispiel C44 | | | 200 | | | 22 | | 11 | 7 | 144 | 9,0 |
| Beispiel C45 | | | 200 | | | 82 | | 41 | 27 | 544 | 22,8 |
| Beispiel C46 | | | 200 | | | 175 | | 88 | 58 | 1168 | 33,5 |
| Beispiel C47 | | | 200 | | | 24 | 4 | 12 | 4 | 160 | 10,5 |

(fortgesetzt)

| | Neutral-pellets | Zitronensäure-Starter | Weinsäure-Starter | Bernsteinsäure-Starter | Fumarsäure-starter | Lefradafiban | Povidone K90 | Talkum | Klucel EF | Isopropanol (nur zur Herstellung) | exp. Wirk-stoffgehalt [%] |
|---|---|---|---|---|---|---|---|---|---|---|---|
| Beispiel C48 | | | 200 | | | 94 | 16 | 47 | 16 | 624 | 24,8 |
| Beispiel C49 | | | 200 | | | 192 | 32 | 96 | 32 | 1280 | 34,7 |
| Beispiel C50 | | 200 | | | | 24 | 8 | 12 | | 160 | 9,8 |
| Beispiel C51 | | 200 | | | | 82 | 27 | 41 | | 544 | 23,4 |
| Beispiel C52 | | 200 | | | | 175 | 58 | 88 | | 1168 | 34,4 |

Tabelle 14:

| | Neutral-pellets | Zitronensäure-Starter | Weinsäure-Starter | Bernsteinsäure-Starter | Fumarsäure-Starter | Amelubant | Povidone K90 | Talkum | Klucel EF | Isopropanol (nur zur Herstellung) | exp. Wirkstoffgehalt |
|---|---|---|---|---|---|---|---|---|---|---|---|
| Beispiel C53 | 200 | | | | | 13 | 3 | 3 | | 104 | 6 |
| Beispiel C54 | | | 200 | | | 23 | 5 | 5 | | 184 | 10 |
| Beispiel C55 | | | 200 | | | 48 | 10 | 10 | | 384 | 18 |
| Beispiel C56 | | | | 200 | | 38 | 8 | 8 | | 304 | 15 |
| Beispiel C57 | | | | | 200 | 33 | 7 | 7 | | 264 | 13 |

Tabelle 15:

| | Neutral-pellets | Zitronensäure-Starter | Weinsäure-Starter | Bernsteinsäure-Starter | Fumarsäure-Starter | Telmisartan | Povidone K90 | Talkum | Klucel EF | Isopropanol (nurzur Herstellung) | exp. Wirkstoffgehalt |
|---|---|---|---|---|---|---|---|---|---|---|---|
| Beispiel C58 | 200 | | | | | 7 | 2 | 3 | | 85 | 3 |
| Beispiel C59 | 200 | | | | | 18 | 4 | 9 | | 220 | 8 |
| Beispiel C60 | | | 200 | | | 7 | 2 | 3 | | 85 | 3 |
| Beispiel C61 | | | 200 | | | 20 | 5 | 10 | | 250 | 9 |

*d: Beispiel für die Isolierung der wirkstoffhaltigen Pellets*

Zusammensetzung:

| | |
|---|---|
| Wirkstoffhaltige Pellets | 23 Gewichtsteile |
| Gummi arabicum | 1 Gewichtsteil |
| Talkum | 2 Gewichtsteile |

In einer Mischung aus 6,7 Gewichtsteilen Ethanol 96 % und 13,5 Gewichtsteilen gereinigtem Wasser wird unter Rühren 1 Gewichtsanteil Gummi arabicum gelöst. Anschließend werden in der Lösung unter Rühren 2 Gewichtsanteile Talkum dispergiert.

In einer Wirbelschichtprozessanlage werden 23 Gewichtanteile wirkstoffhaltige Pellets bei einer Zulufttemperatur von 35° - 40° C mit der Gummi arabicum-Talkum - Dispersion im Unterbettsprühverfahren besprüht.

Das isolierte weinsäurehaltige Kernmaterial wird anschließend im Umlufttrockenschrank bei 40° C über 8 Stunden nachgetrocknet.

Zur Entfernung von Agglomeraten werden die getrockneten wirkstoffhaltigen Pellets mit einem Sieb mit der nominalen Maschenweite 1,25mm gesiebt. Die Gutfraktion (Korngröße < 1.25 mm) wird weiterverarbeitet.

*e) Abfüllung in Kapseln*

Eine jeweils der gewünschten Dosierung entsprechende Menge an wirkstoffhaltigen Pellets wird mittels einer Kapselfüllmaschine in Hartgelatinekapseln einer geeigneten Größe abgefüllt.

Gegenstand der Erfindung ist eine pharmazeutische Zusammensetzung mit einer vom Magen-pH weitgehend unabhängigen Bioverfügbarkeit des Wirkstoffs zur oralen Verabreichung von Wirkstoffen mit pH-abhängigen Löslichkeiten und einer Dosenumber größer 1 bei pH > 5 umfassend eine Vielzahl von Pellets, die jeweils aus

a) einem Kernmaterial,
b) einer optionalen Isolierschicht,
c) einer Wirkstoffschicht und
d) einem optionalem Überzug

aufgebaut sind, dadurch gekennzeichnet, daß das Kernmaterial aus einer oder mehreren pharmazeutisch akzeptablen organischen Säure(n) mit einer Wasserlöslichkeit von > 1 g / 250 ml bei 20° C, gegebenenfalls unter Zusatz von Bindemitteln oder anderer technologischer Hilfsstoffe, besteht.

Ein zweiter Erfindungsgegenstand ist eine pharmazeutische Zusammensetzung gemäß obiger Definition, in der der Wirkstoff Lefradafiban, (R)-2-[4-(N-Phenylcarbonylamidino)-phenylaminomethyl]-1-methyl-5-[1-(n-propyloxycarbonylmethylamino)-1-(pyrrolidinocarbonyl)-ethyl]-benzimidazol, Telmisartan, DTTX 30 SE, Terbogrel, Amelubant, Flibanserin , 4-(4-(2-Pyrrolylcarbonyl)-1-piperazinyl)-3-trifluormethyl-benzoylguanidin oder Pimobendan oder eines der pharmakologisch verträglichen Salze dieser Verbindungen ist. Bevorzugt sind Flibanserin, Telmisartan und Pimobendan oder eines der pharmakologisch verträglichen Salze diese Verbindungen.

Geeignete pharmazeutisch akzeptable organische Säuren für die erfindungsgemäßen pharmazeutischen Zusammensetzungen sind Weinsäure, Fumarsäure, Bernsteinsäure, Zitronensäure, Äpfelsäure, Glutaminsäure und Asparaginsäure oder eines von deren Hydraten oder sauren Salzen; vorzugsweise Weinsäure, Fumarsäure, Zitronensäure oder Bernsteinsäure, insbesondere jedoch Weinsäure.

Geeignete Bindemittel für die erfindungsgemäßen pharmazeutischen Zusammensetzungen sind Hydroxypropylcellulose, Hydroxypropylmethylcellulose, Methylcellulose, Hydroxyethylcellulose, Carboxymethylcellulose, Polyvinylpyrrolidon, Copolymerisate aus N-Vinylpyrrolidon und Vinylacetat oder Kombinationen dieser Polymere.

Das Kernmaterial der erfindungsgemäßen pharmazeutischen Zusammensetzungen weist vorzugsweise eine mittlere Partikelgröße von 0,4 bis 1,5 mm auf.

Vorzugsweise besteht die Isolierschicht der erfindungsgemäßen pharmazeutischen Zusammensetzungen aus einem wasserlöslichen Polymer, ggf. unter Zusatz geeigneter Weichmacher, Trennmittel und Pigmente. Dabei sind folgende wasserlösliche Polymere bevorzugt: Gummi arabicum oder ein partial- oder vollsynthetisches Polymer aus der Gruppe der Hydroxypropylcellulosen, der Hydroxypropylmethylcellulosen, der Methylcellulosen, der Hydroxyethylcellulosen, der Carboxymethylcellulosen, der Polyvinylpyrrolidone, der Copolymerisate aus N-Vinylpyrrolidon und Vinylacetat, sowie Kombinationen dieser Polymere.

Vorzugsweise besteht der Überzug der erfindungsgemäßen pharmazeutischen Zusammensetzungen aus Filmbildnern, Weichmachern und ggf. Pigmenten.

Vorzugsweise sind solche erfindungsgemäßen pharmazeutischen Zusammensetzungen, bei denen die wirkstoffhaltige Pellets in Hartkapseln abgefüllt werden.

## Patentansprüche

1. Pharmazeutische Zusammensetzung mit einer vom Magen-pH weitgehend unabhängigen Bioverfügbarkeit des Wirkstoffs zur oralen Verabreichung von Flibanserin oder eines seiner pharmakologisch verträglichen Salze umfassend eine Vielzahl von Pellets, die jeweils aus

    a) einem Kernmaterial,
    b) einer optionalen Isolierschicht,
    c) einer Wirkstoffschicht und
    d) einem optionalem Überzug

    aufgebaut sind, **dadurch gekennzeichnet, daß** das Kernmaterial aus einer oder mehreren pharmazeutisch akzeptablen organischen Säure(n) mit einer Wasserlöslichkeit von > 1 g / 250 ml bei 20° C, gegebenenfalls unter Zusatz von Bindemitteln oder anderer technologischer Hilfsstoffe, besteht.

2. Pharmazeutische Zusammensetzung nach Anspruch 1, bei denen die pharmazeutisch akzeptable organische Säure Weinsäure, Fumarsäure, Bernsteinsäure, Zitronensäure, Äpfelsäure, Glutaminsäure und Asparaginsäure oder eines von deren Hydraten oder sauren Salzen ist.

3. Pharmazeutische Zusammensetzung nach Anspruch 2, **dadurch gekennzeichnet, dass** die pharmazeutisch akzeptable organische Säure Weinsäure, Fumarsäure, Zitronensäure oder Bernsteinsäure ist.

4. Pharmazeutische Zusammensetzung nach Anspruch 2, **dadurch gekennzeichnet, dass** die pharmazeutisch akzeptable organische Säure Weinsäure ist.

5. Pharmazeutische Zusammensetzung nach einem der vorangehenden Ansprüche, bei der das Bindemittel aus der Gruppe der Hydroxypropylcellulosen, der Hydroxypropylmethylcellulosen, der Methylcellulosen, der Hydroxyethylcellulosen, der Carboxymethylcellulosen, der Polyvinylpyrrolidone, der Copolymerisate aus N-Vinylpyrrolidon und Vinylacetat oder aus Kombinationen dieser Polymere besteht.

6. Pharmazeutische Zusammensetzung nach einem der vorangehenden Ansprüche, bei der das Kernmaterial eine mittlere Partikelgröße von 0,4 bis 1,5 mm aufweist.

7. Pharmazeutische Zusammensetzung nach Anspruch 1, bei der die Isolierschicht aus einem wasserlöslichen Polymer, ggf. unter Zusatz geeigneter Weichmacher, Trennmittel und Pigmente, besteht.

8. Pharmazeutische Zusammensetzung nach Anspruch 7, bei der das wasserlösliche Polymer aus Gummi arabicum oder einem partial- oder vollsynthetischem Polymer aus der Gruppe der Hydroxypropylcellulosen, der Hydroxypropylmethylcellulosen, der Methylcellulosen, der Hydroxyethylcellulosen, der Carboxymethylcellulosen, der Polyvinylpyrrolidone, der Copolymerisate aus N-Vinylpyrrolidon und Vinylacetat, oder aus Kombinationen dieser Polymere besteht.

9. Pharmazeutische Zusammensetzung nach Anspruch 1, bei der der Überzug aus Filmbildnern, Weichmachern und ggf. Pigmenten besteht.

10. Pharmazeutische Zusammensetzung nach einem der vorangehenden Ansprüche, bei der die wirkstoffhaltige Pellets in Hartkapseln abgefüllt werden.

11. Verfahren zur Herstellung einer oral zu applizierenden pharmazeutischen Zusammensetzung gemäß einem der vorangehenden Ansprüche, umfassend die Schritte:

    a) Aufbau des Kernmaterials aus einer oder mehreren pharmazeutisch akzeptablen organischen Säure(n) mit einer Wasserlöslichkeit von > 1 g / 250 ml bei 20° C, gegebenenfalls unter Zusatz von Bindemitteln oder anderer technologischer Hilfsstoffe, in Kesselverfahren, auf Polletiertellern oder mittels Extrusion /Sphäronisierung,

b) Auftragen einer Isolierschicht bestehend aus einem oder mehreren wasserlöslichen, pharmazeutisch akzeptablen Polymeren gegebenenfalls unter Zusatz von Weichmachern, Trennmitteln und/oder Pigmenten auf das Kernmaterial,

c) Auftragen des Wirkstoffs aus einer bindemittel- und gegebenenfalls trennmittelhaltigen Dispersion und gleichzeitiges und/oder anschließendes Trocknen zur Entfernung des Dispersionsmittels,

d) gegebenenfalls Aufbringen eines Überzugs aus Filmbildnern, Weichmachern und gegebenenfalls Pigmenten und

e) Abfüllung der so erhaltenen wirkstoffhaltigen Pellets in Hartkapseln.

12. Pharmazeutische Zusammensetzung mit einer vom Magen-pH weitgehend unabhängigen Bioverfügbarkeit des Wirkstoffs zur oralen Verabreichung von Wirkstoffen mit pH-abhängigen Löslichkeiten und einer Dosenumber größer 1 bei pH > 5 umfassend eine Vielzahl von Pellets, die jeweils aus

a) einem Kernmaterial,

b) einer optionalen Isolierschicht,

c) einer Wirkstoffschicht und

d) einem optionalem Überzug

aufgebaut sind, **dadurch gekennzeichnet, daß** das Kernmaterial aus einer oder mehreren pharmazeutisch akzeptablen organischen Säure(n) mit einer Wasserlöslichkeit von > 1 g / 250 ml bei 20° C, gegebenenfalls unter Zusatz von Bindemitteln oder anderer technologischer Hilfsstoffe, besteht.

13. Pharmazeutische Zusammensetzung gemäß Anspruch 12, wobei der Wirkstoff Lefradafiban, (*R*)-2-[4-(*N*-Phenylcarbonylamidino)-phenylaminomethyl]- 1- methyl- 5-[1-(*n*-propyloxycarbonylmethylamino)- 1-(pyrrolidinocarbonyl)-ethyl]-benzimidazol, Telmisartan, DTTX 30 SE, Terbogrel, Amelubant, Flibanserin, 4-(4-(2-Pyrrolylcarbonyl)-1-piperazinyl)-3-trifluormethyl-benzoylguanidin, Pimobendan oder eines der pharmakologisch verträglichen Salze dieser Verbindungen ist.

14. Pharmazeutische Zusammensetzung nach Anspruch 12 oder 13, bei der die pharmazeutisch akzeptable organische Säure Weinsäure, Fumarsäure, Bernsteinsäure, Zitronensäure, Äpfelsäure, Glutaminsäure und Asparaginsäure oder eines von deren Hydraten oder sauren Salzen ist.

15. Pharmazeutische Zusammensetzung nach Anspruch 14, **dadurch gekennzeichnet, dass** die pharmazeutisch akzeptable organische Säure Weinsäure, Fumarsäure, Zitronensäure oder Bernsteinsäure ist.

16. Pharmazeutische Zusammensetzung nach Anspruch 14, **dadurch gekennzeichnet, dass** die pharmazeutisch akzeptable organische Säure Weinsäure ist.

17. Pharmazeutische Zusammensetzung nach einem der Ansprüche 12 bis 16, bei der das Bindemittel aus der Gruppe der Hydroxypropylcellulosen, der Hydroxypropylmethylcellulosen, der Methylcellulosen, der Hydroxyethylcellulosen, der Carboxymethylcellulosen, der Polyvinylpyrrolidone, der Copolymerisate aus N-Vinylpyrrolidon und Vinylacetat oder aus Kombinationen dieser Polymere besteht.

18. Pharmazeutische Zusammensetzung nach einem der Ansprüche 12 bis 17, bei der das Kernmaterial eine mittlere Partikelgröße von 0,4 bis 1,5 mm aufweist.

19. Pharmazeutische Zusammensetzung nach Anspruch 12 oder 13, bei der die Isolierschicht aus einem wasserlöslichen Polymer, ggf. unter Zusatz geeigneter Weichmacher, Trennmittel und Pigmente, besteht.

20. Pharmazeutische Zusammensetzung nach Anspruch 19, bei der das wasserlösliche Polymer aus Gummi arabicum oder einem partial- oder vollsynthetischem Polymer aus der Gruppe der Hydroxypropylcellulosen, der Hydroxypropylmethylcellulosen, der Methylcellulosen, der Hydroxyethylcellulosen, der Carboxymethylcellulosen, der Polyvinylpyrrolidone, der Copolymerisate aus N-Vinylpyrrolidon und Vinylacetat, oder aus Kombinationen dieser Polymere besteht.

21. Pharmazeutische Zusammensetzung nach Anspruch 12 oder 13, bei der der Überzug aus Filmbildnern, Weichmachern und ggf. Pigmenten besteht.

**22.** Pharmazeutische Zusammensetzung nach einem der Ansprüche 12 bis 21, bei der die wirkstoffhaltige Pellets in Hartkapseln abgefüllt werden.

**23.** Verfahren zur Herstellung einer oral zu applizierenden pharmazeutischen Zusammensetzung enthaltend einen Wirkstoff mit pH-abhängigen Löslichkeitsverhalten und einer Dosenumber >> 1 bei pH > 5 oder eines seiner physiologisch verträglichen Salze, umfassend die Schritte:

a) Aufbau des Kernmaterials aus einer oder mehreren pharmazeutisch akzeptablen organischen Säure(n) mit einer Wasserlöslichkeit van > 1 g / 250 ml bei 20° C, gegebenenfalls unter Zusatz von Bindemitteln oder anderer technologischer Hilfsstoffe, in Kesselverfahren, auf Pelletiertellern oder mittels Extrusion /Sphäronisierung,
b) Auftragen einer Isolierschicht bestehend aus einem oder mehreren wasserlöslichen, pharmazeutisch akzeptablen Polymeren gegebenenfalls unter Zusatz von Weichmachern, Trennmitteln und/oder Pigmenten auf das Kernmaterial,
c) Auftragen des Wirkstoffs aus einer bindemittel- und gegebenenfalls trennmittelhaltigen Dispersion und gleichzeitiges und/oder anschließendes Trocknen zur Entfernung des Dispersionsmittels,
d) gegebenenfalls Aufbringen eines Überzugs aus Filmbildnern, Weichmachern und gegebenenfalls Pigmenten und
e) Abfüllung der so erhaltenen wirkstoffhaltigen Pellets in Hartkapseln.

Figur 1:

**Schematischer Aufbau der pharmazeutischen Zusammensetzung:**

Kernmaterial
enthaltend organische Säure

Isolierschicht (optional)

Wirkstoffschicht

Überzug (optional)

Figur 2:

Bioverfügbarkeit von BIBR 1048

Figur 3:

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9837075 A
- EP 0483667 A
- WO 0147896 A
- WO 9602497 A
- EP 526434 A
- WO 0017176 A

**In der Beschreibung aufgeführte Nicht-Patentliteratur**

- **LÖBENBERG, R. ; AMIDON, G.L.** Modern bioavailability, bioequivalence and biopharmaceutics classification system. New scientific approaches to international regulatory standards. *Eur. J. Pharm. Biopharm.,* 2000, vol. 50, 3-12